(19)

Europäisches
Patentamt

European
Patent Office

Office européen
des brevets

(11) **EP 2 774 655 A1**

(12) **EUROPEAN PATENT APPLICATION**

(43) Date of publication:
**10.09.2014 Bulletin 2014/37**

(21) Application number: **13157848.6**

(22) Date of filing: **05.03.2013**

(51) Int Cl.:
*A61Q 19/10* (2006.01)          *A61K 8/98* (2006.01)
*C09K 3/14* (2006.01)          *C11D 3/12* (2006.01)
*C11D 3/14* (2006.01)          *A61K 8/73* (2006.01)
*A61K 8/81* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
• **JAO beheer BV**
  **6097 CR Heel (NL)**
• **Beckman Lapre Beheer BV**
  **8621 CV Heeg (NL)**

• **Schaffelaarbos BV**
  **3771 MD Barneveld (NL)**

(72) Inventor: **Brand-Garnys, Elzbieta Ewa**
  **2914 AD Nieuwerkerk aan den IJssel (NL)**

(74) Representative: **Farago, Peter Andreas et al**
  **Schieber Farago**
  **Thierschstrasse 11**
  **80538 München (DE)**

(54) **Composition comprising chicken eggshell particles, preparation and use**

(57)     The present invention relates to composition comprising chicken egg shell particles. We have surprisingly found that chicken egg particles can be advantageously used in treatment of the skin. Other applications include hard surface cleaning. The invention relates to compositions, as well as methods of preparing and applications of the chicken egg particles.

EP 2 774 655 A1

**Description**

**Technical field**

The present invention relates to compositions, to a method of preparing a composition, to a method of treating the skin, to a method of preparing particles, to a method of preparing a composition, and to the use of particles.

**Background to the invention**

Various types of particulate material have been used for scrub and abrasive compositions including polyethylene particles. Recently, however, it has been concluded that these plastic particles accumulate in the environment and represent a significant aquatic health hazard. Alternative scrub compositions have been proposed and are used, such as formulations comprising bamboo fibres, crushed almond and walnut shells, coral & sea lichen, loofah, and other hard particles from vegetable origin. These alternatives may, however, be associated with difficulties of sourcing, difficulties in processing, dermatological limitations and complications in creating stable products. Products containing these substances are often unstable. Particles may be sharp to the skin, potentially even leading to skin injuries. The resulting products may not have customer preferred properties for instance with particles that are too hard, too soft, too large or too small or sometimes with particles that have undesired changing character during storage or usage.

US2005/0170979 (Unilever) discloses liquid detergent compositions that are internally structured using lamellar droplets of surfactant material and have an acidic pH. US2003/0133900 (McLaughlin) refers to powdered eggshell and discloses pastes and creams containing them.

Described formulations may show chemically and/or physically instability and often provide limited customer benefits, particularly after storage.

**Summary of the invention**

The present invention overcomes one or more of the above mentioned or other problems. Accordingly, the present invention relates to a composition comprising from 1 to 30% by weight of chicken eggshell particles wherein at least 50% by weight of the chicken eggshell particles has a particle size of at least 74$\mu$m and at most 595$\mu$m. Preferably, the pH of the composition is higher than 7.5 and lower than 9 and wherein the composition further comprises a polymer providing an external structure to the composition. Preferably, the composition further comprises from 1-20% by weight of anionic surfactant and from 0.5-12% by weight of non-ionic surfactant with a ratio of anionic to nonionic surfactant of from 3:1 to 4:1. Preferably, the composition comprises egg shell membrane particles. Preferably, the composition further comprises water at a level of from 2 to 90% by weight of composition. Preferably, the composition further comprises emollient at a level of from 0.2% to 40% by weight of the composition.

The invention further relates to a method of preparing a composition comprising chicken eggshells and polymer material, wherein the chicken eggshell is ball-milled to particles which are subsequently dispersed in the composition comprising polymer material and wherein the pH is subsequently set at higher than 7.5. Preferably, at least 50% by weight of the chicken eggshell particles has a particle size of at least 74$\mu$m and at most 595$\mu$m.

The invention further relates to a method of treating the skin by removing dead skin cells from the skin surface with chicken eggshell particles. Preferably, the chicken eggshell particles are first ball-milled. Preferably, the skin treatment comprises the steps of (a) providing a cleaning composition comprising surfactant and chicken eggshell particles; (b) applying the composition to the skin;(c) adding water to wet the skin and develop a lather; and (d) rubbing the lather into the skin to remove dead skin cells from the skin surface.

The invention further relates to a method of preparing chicken eggshell particles by washing and ball-milling the eggshells, wind sifting and sterilizing the resulting chicken eggshell particles, and subsequently sieving the chicken eggshell particles into fractions. The invention further relates to a method of preparing a composition comprising chicken eggshell particles dispersed in an aqueous composition comprising polymer, wherein the polymer is mixed with water, the chicken eggshell particles are added under stirring and the pH of the composition is set at higher than 7.5.

The invention further relates to chicken eggshell particles for use in treatment of skin. Preferably, at least 50% by weight of the chicken eggshell particles has a particle size of at least 74$\mu$m and at most 595$\mu$m.

The present invention surprisingly provides for chemically and physically stable compositions with chicken eggshells that offer preferred properties, even after prolonged storage at elevated temperature and without detrimental effects on the environment. The invention offers a low cost option with abundant supply (in 2011 about 450,000 tonnes) and relatively easy processing.

Surprisingly, we have found that chicken eggshell particles can be chemically and physically stably suspended in compositions of the present invention. The chicken eggshell particles do not accumulate in the environment and are fully biodegradable, are of extreme purity, are compatible with other ingredients present in compositions, absence of undesirable contaminants, are easy to process and provide excellent scrubbing benefits. According to the invention, chicken eggshell particles are beneficially applied to remove dead skin cells from the skin surface. According to the invention, larger particles of chicken eggshell are preferably used for scrubbing the skin, while smaller particles of chicken eggshell are preferably used for polishing the skin.

In a further embodiment, chicken eggshell particles of the invention are preferably used as abrasive in tooth paste. In a further embodiment, chicken eggshell particles of the invention are preferably used as abrasive in hard surface cleaners. In a further embodiment, chicken eggshell particles of the invention are preferably used as abrasive in car polish. In a further embodiment, chicken eggshell particles of the invention are preferably used as filler for surface coatings. In a further embodiment, chicken eggshell particles of the invention are preferably used as tableting aids in pharmaceutical compositions. In a further embodiment, chicken eggshell particles of the invention are preferably used as antacids.

## Detailed description of the invention

Compositions according to the invention comprise chicken eggshell particles. Chicken eggshell particles are preferably used in personal care products, cosmetic cream products (both scrub and polish), in household products, in chemical technical products and in medical products.

In its non-processed form, a chicken eggshell consists mostly of calcite, a crystal form of calcium carbonate. It contains substantially no toxins. Generally between 95-97% of chicken eggshells represents calcium carbonate. The structure of the chicken eggshell of the invention is porous yet hard, making chicken eggshell particles particularly suitable for use in the compositions of the present invention. Furthermore, calcium carbonate originating from chicken egg shells have high purity (contains only trace amounts if any), enabling to usefully apply chicken egg shells as food supplement or as food additive to avoid calcium deficiency in adults, which can eventually lead to osteoarthritis and osteoporosis.

We have surprisingly found that compositions of the invention comprising chicken eggshell particles enable removal of dead cells from the stratum corneum. Not being bound by theory, the degree thereof depends on the size and morphology of the particles, and their hardness. Large, rough and hard particles are usually harsh and irritating to the skin, while small and soft particles may not be less effective. The abrasive properties are also determined by the hardness of the chicken eggshell particles. Hardness is typically expressed using the Moh scale. Moh's scale of mineral hardness characterises the scratch resistance of various minerals through the ability of a harder material to scratch a softer material. The scale ranges in order of increasing relative hardness from 1 (softest, e.g. talc) to 10 (hardest, e.g. diamond). Preferably, the Moh hardness of the chicken eggshell particles of the invention is below 5 and preferably at least 3, and best between 3.5 and 4 (absolute hardness 10-20 using a sclerometer).

Chicken eggshell particles are distinct from calcium carbonate particles from other sources, such as mined calcium carbonate, precipitated calcium carbonate and other eggshell particles. In comparison, mined calcium carbonate contains undesirable concentration of elements (sodium, magnesium and heavy metals/transition elements (strontium [ up to 1% in aragonite], barium, lead, copper, zinc, strontium, uranium, manganese, iron), due to its mineral origin. Precipitated calcium carbonate may contain undesirable sharp edged particles, possibly due to the crystallization step involved. Other sources for eggshells include quail eggshells, ostrich eggshells and in particular emu eggshells which also have abundant supply. However, these eggshells are more difficult to process and do not provide the benefits of the chicken egg particles by the present invention.

Surprisingly, we have found that chicken eggshell particles can be stably incorporated in compositions of the invention, largely eliminating sharp edges, and providing safe products with low or no contamination and with preferred processing and user characteristics even after storage of said compositions.

Further, surprisingly, upon application and use of the compositions of the invention, for instance on the skin, the chicken egg shell particles may further slowly lose their sharp edges, possibly due to their porosity. Consequently, for skin applications the efficacy of removing dead cells from the skin surface seems to slowly decrease during usage of the chicken eggshell particles in compositions of the invention, presenting a unique benefit, not provided by the other agents mentioned.

According to the invention, the chicken eggshell particle size is subdivided in different fractions using sieves according to ASTM C136-06. These sieves have different mesh sizes including the following 1000$\mu$m (mesh 18), 841$\mu$m (mesh 20), 707$\mu$m (mesh 25), 595$\mu$m (mesh 30), 500$\mu$m (mesh 35), 420$\mu$m (mesh 40), 354$\mu$m (mesh 45), 297$\mu$m (mesh 50), 250$\mu$m (mesh 60), 210$\mu$m (mesh 70), 177$\mu$m (mesh 80), 149$\mu$m (mesh 100), 25$\mu$m (mesh 120), 105$\mu$m (mesh 140), 88$\mu$m (mesh 170), 74$\mu$m (mesh 200), 63$\mu$m (mesh 230), 53$\mu$m (mesh 270), 44$\mu$m (mesh 325), and 37$\mu$m (mesh 400). Also smaller fraction sizes are possible. By sieving particles, the preferred particle size can be obtained. According to the invention, the particle size is determined by means of sieve analysis using standardised sieves according to ASTM C136-06. In line with the above sieving method, particle size determination according to the invention is based on the smallest diameter (other options in the art include particle size determination based on volume or surface area).

Equipment to determine the particle size according to the invention in products, such as dispersions that falls within the scope of the present invention, is available in the art. For instance, Malvern equipment is suitable, but so are similar devices available elsewhere.

Preferably, at least 50% by weight of chicken eggshell particles in the composition of the invention has a particle size of preferably at least 74μm, more preferably at least 88μm, most preferably at least 125μm and in particularly preferred at least 149μm and preferably at most to 595μm, more preferably at most 500μm, most preferably at most 420μm. More preferably, at least 65% by weight of the chicken eggshell particles has such size, most preferably at least 80%, and in particular preferred at least 95% by weight of the chicken eggshell particles.

Preferably, at least 50% by weight of chicken eggshell particles in compositions for scrubbing has a particle size of preferably at least 149μm, more preferably at least 177μm, most preferably at least 210μm and preferably at most to 595μm, more preferably at most 500μm, most preferably at most 420μm. More preferably, at least 65% by weight of the chicken eggshell particles has such size, most preferably at least 80%, and in particular preferred at least 95% by weight of the chicken eggshell particles.

Preferably, at least 50% by weight of chicken eggshell particles in compositions for polishing and for hard surface cleaning has a particle size of preferably at least 74μm, more preferably at least 88μm, most preferably at least 125μm and in particularly preferred at least 149μm and preferably at most 354μm, more preferably at most 297μm, most preferably at most 210μm. More preferably, at least 65% by weight of the chicken eggshell particles has such size, most preferably at least 80%, and in particular preferred at least 95% by weight of the chicken eggshell particles.

Preferably, compositions of the invention comprise chicken eggshell particles at a level of at least 1% by weight, more preferably at least 2% by weight, most preferably at least 3% by weight of the composition and preferably at most 30% by weight, more preferably at most 20% by weight, most preferably at most 15% by weight and in particular preferred at most 7% by weight of the composition.

In a preferred embodiment, personal care scrub compositions and cosmetic cream compositions preferably comprise chicken eggshell particles at a level at least 1% by weight, more preferably at least 2% by weight, most preferably at least 3% by weight of the composition and preferably at most 12% by weight, more preferably at most 8% by weight, most preferably at most 6% by weight and in particular preferred at most 5% by weight of the composition.

In a preferred embodiment, household compositions, and preferably hard surface cleaner compositions, comprise eggshell particles preferably at a level at least 1% by weight, more preferably at least 3% by weight, most preferably at least 4% by weight of the composition and preferably at most 30% by weight, more preferably at most 20% by weight, most preferably at most 15% by weight and in particular preferred at most 7% by weight of the composition.

Chicken eggshell is covered on the inside with a separate membrane, often named the eggshell membrane. For the purpose of this invention, reference to chicken eggshell refers to the outer shell only and does not include this membrane. Chicken eggshell particles of the invention may contain some membrane as well, but preferably comprises at least 90% of calcium carbonate. The eggshell membrane is composed of collagen and mainly consists of collagen type I, and has an $\alpha$1 chain (<MW>=130 kDa) and $\alpha$2 chain (<MW>=116 kDa). The ratio $\alpha$1/$\alpha$2 is approximately 1 (Zhao, Chi, Biotechnology 8(2):254-258,2009). Eggshell membrane further contains significant amounts of glucosamine, chondroitin sulphate and hyaluronic acid. We have found that presence of these constituents of the eggshell membrane is beneficial in consumer products such as bath & shower products for skin scrubbing. According to the invention, the eggshell membrane is preferably at least partially separated from the eggshell. This can for instance be accomplished by treatment with hot/boiling water and/or by wind sifting as will be explained below. JP4169513 and JP4187616 describe separating the membrane from the eggshell but dissolve the eggshell in lactic acid. The membrane is subsequently used for cosmetic skin purposes.

In a preferred embodiment of the present invention, eggshell membrane particles are prepared from eggshell membrane. These membrane particles comprise of at least 90% by weight of eggshell membrane. The membrane particles may be added to the composition with the chicken eggshell particles and, this way, a well-defined egg shell fraction is obtained for beneficial application in personal care and/or cosmetic consumer products, and in particular for skin treatment. Thus, the present invention further relates to a composition comprising chicken eggshell particles and eggshell membrane particles.

Preferably, at least 50% of eggshell membrane particles in the composition of the invention has a particle size of at least 149μm, more preferably at least 177μm, most preferably at least 210μm and preferably at most to 595μm, more preferably at most 500μm, most preferably at most 420μm. More preferably, at least 65% by weight of the eggshell membrane particles has such size, most preferably at least 80% by weight, and in particular preferred at least 95% by weight of the chicken eggshell particles.

Preferably, the composition of the invention comprises eggshell membrane particles at a level of at least 0.1 % by weight and preferably at most 20% by weight of the composition.

Preferably, compositions of the invention comprise one or more surfactants, which are sometimes called surface active agents. In general, surfactant may be chosen from the surfactants described in well-known textbooks like "Surface Active Agents" Vol. 1, by Schwartz & Perry, Interscience 1949, Vol. 2 by Schwartz, Perry & Berch, Interscience 1958, and/or the current edition of "McCutcheon's Emulsifiers and Detergents" published by Manufacturing Confectioners Company or in "Tenside-Taschenbuch" , H. Stache, 2nd Edn., Carl Hauser Verlag, 1981 and "Surfactant Encyclopaedia, 2nd edition, by Rieger (Allured Publishing, 1996), and other sources from the open literature. Any type of surfactant, i.e. anionic, cationic, nonionic or amphoteric surfactant, can be used. Preferably, the composition comprises anionic and/or nonionic surfactant.

Preferred anionic surfactants are water-soluble salts of organic sulphuric acid esters and sulphonic acids having in the molecular structure an alkyl group containing 8-22 carbon atoms or an alkylaryl group containing 6-20 C atoms in the alkyl part. Examples of such anionic surfactants are water soluble salts of:

- long chain (i.e. 8-22 carbon atom) alcohol sulphates (hereinafter referred to as PAS), especially those obtained by sulphating the fatty alcohols obtained from vegetable oils by (1) trans-esterification of the triglyceride, and (2) followed by catalytic reduction with hydrogen. The counter-ion may be sodium, potassium, calcium, magnesium, ammonium or zinc ions, or alkanolamines such as but not limited to monoethanolamine, triethanolamine and triisopropanolamine. Suitable vegetable oils are coconut and palm kernel oil;
- secondary alkyl sulphonates derived from unsaturated petrochemical feedstock, using as the counter-ion sodium, potassium, calcium, magnesium, ammonium or zinc, or alkanolamines such as but not limited to monoethanolamine, triethanolamine and triisopropylamine.
- alkylbenzene sulphonates, such as those in which the alkyl group contains from 6 to 20 carbon atoms using as the counter-ion sodium, potassium, calcium, magnesium, ammonium or zinc, or alkanolamines such as but not limited to monoethanolamine, triethanolamine and triisopropylamine;

Also suitable anionics are the salts of:

- sulphates of ethoxylated aliphatic alcohols containing 1-12 alkylene oxide units in which the alkylene group contains from 2 to 14 carbon atoms;
- ethoxylated alkylphenols sulphates with from 1 to 30 alkylene oxide units in which the alkyl group contains from 2 to 14 carbon atoms;
- sarcosinates, preferably as the ammonium or triethanolamine salt;
- taurates, preferably as the sodium salt;
- ester carboxylates derived from di- or tricarboxylic acid such as citric acid & ethercarboxylates derived from ethoxylated fatty alcohols with 2-20 ethylene oxide units and reacted with chloroacetic acid;
- sulfosuccinates derived from maleic acid anhydride, reacted with a suitable fatty alcohol or ethoxylated fatty alcohol, followed by sulphonation and neutralisation with a suitable anion;
- acylamino acids (acylglutamates, as mono- or disodium salts).

As the counter-ion may be used sodium, potassium, calcium, magnesium, ammonium or zinc, or alkanolamines such as -but not limited to- monoethanolamine, triethanolamine and triisopropylamine. Preferably anionic surfactants are used that do not form insoluble salts with divalent metal ions, such as calcium & magnesium.

Suitable nonionic surfactants can be broadly described as compounds produced by the condensation of simple alkylene oxides, which are hydrophilic in nature, with an organic hydrophobic compound which may be aliphatic or alkyl-aromatic in nature. The length of the hydrophilic or polyoxyalkylene chain which is attached to any particular hydrophobic group can be readily adjusted to yield a water-soluble compound having the desired balance between hydrophilic and hydrophobic elements. This enables the choice of nonionic surfactants with the right HLB.

Particular examples include the condensation products of aliphatic alcohols having from 8 to 22 carbon atoms in either straight or branched chain configuration with ethylene oxide, such as a coconut alcohol ethylene oxide condensates having from 2 to 15 moles of ethylene oxide per mole of fatty alcohol; condensates of alkylphenols having C6-C15 alkyl groups with 5 to 25 moles of ethylene oxide per mole of alkylphenol; condensates of the reaction product of ethylenediamine and propylene oxide with ethylene oxide, the condensates containing from 40 to 80% of oxyethylene groups by weight.

Petrochemical alcohols with 7-25 carbon atoms, reacted with alkylene oxide (2-20 units), are also suitable nonionic surfactants. These alkoxylated long chain alcohols may also be converted in the corresponding sulphate, neutralised with a suitable counter-ion. Reaction of the alkoxylated alcohol with chloroacetic acid leads to the formation of ethercarboxylic acids that are suitable as anionic surfactant after neutralisation with sodium hydroxide or triethanolamine.

Other examples are: alkyl polyglucosides, which are condensation products of fatty alcohols and polyglucose obtained from corn; tertiary amine oxides of structure RRRNO, where one R is an alkyl group of 8 to 20 carbon atoms and the other R's are each alkyl or hydroxyalkyl groups of 1 to 3 carbon atoms, e.g. dimethyldodecylamine oxide and fatty acid alkanolamides. Ethoxylated aliphatic alcohols are particularly preferred.

Specific nonionic surfactants are block copolymers of ethylene and propylene oxide and are most suitable in low foaming but effective cleansing preparations. Preferred examples are poloxamines and poloxamers $(POE)_n$-$(PPO)_m$-$(POE)_n$ wherein n=10-200 and m=20-80; a number of poloxamers are fluid (with n=low and m=high) or pasty, some are solid (with n=high and m=moderately low).

Suitable amphoteric surfactants are derivatives of fatty acids having an aliphatic group of from 8 to 18 carbon atoms that have reacted with a suitable diamine such as N,N-dimethyl-1,3-propylenediamine, subsequently reacted with chloroacetic acid to obtain the corresponding zwitterionic species, also named betaines. Other suitable amphoteric surfactants are sultaines and imidazole-based surfactants.

Preferably, compositions of the present invention comprise compositions containing surfactants preferably at least 1% by weight, more preferably at least 2% by weight, most preferably at least 4% by weight of the composition and preferably at most 30% by weight, more preferably at most 20% by weight, most preferably at most 16% by weight of the composition, and in particular preferred around 12% by weight.

Preferably, compositions of the present invention comprise anionic surfactants and preferably at a level of at least 0.5% by weight, more preferably at least 1% by weight, most preferably at least 2% by weight of the composition and preferably at most 20% by weight, more preferably at most 14% by weight, most preferably at most 10% by weight of the composition.

Preferably, compositions of the present invention comprise nonionic surfactants and preferably at a level of at least 0.5% by weight, more preferably at least 1% by weight, most preferably at least 2.5% by weight of the composition and preferably at most 12% by weight, more preferably at most 10% by weight, most preferably at most 6% by weight of the composition.

Preferably, compositions of the present invention comprise anionic and nonionic surfactants and preferably in a ratio of from 3:1 to 4:1.

We have surprisingly found that chicken eggshell particles may be stably incorporated in compositions of the present invention by using particular polymers that provide an external structure to the composition. Preferably, the polymer has a sufficiently high yield stress value. The yield stress value is best defined using the empirical formula by Herschel & Bulkley:

$$\tau = \tau_0 + \alpha x exp(-\beta x D)$$

$\tau$ is the shear stress (expressed in [Pascal) and $\tau_0$ represents the yield stress value, also expressed in [Pa]scal. The constants $\alpha$ and $\beta$ are material parameters characteristic for the product. D represents the shear rate, and is the running parameter, and is expressed in $s^{-1}$.

Preferably, the polymer as rheological additive is selected from different polymer families including (1) acrylic/methacrylic acid homo- & copolymers, (2) polysaccharides obtained from vegetable resources, and mixtures thereof.

Suitable polymers from the group of acrylic/methacrylic acid homo- and copolymers are low cross-linked homopolymers of acrylic acid, better known as carbomer 941 types such as commercially available from Lubrizol, RITA Corporation, Evonik Industries, 3-V Chemical, Guangzhou Tinci Materials Technology Sumitomo Seika Chemicals Co., Nihon Junyaku, and others. Copolymers of acrylic & methacrylic acid are known as acrylates/C10-30 alkyl acrylate copolymer, commercially available from Evonik Industries, Lubrizol, Rita Corporation, Sumitomo Seika Chemicals Co., Guangzhou Tinci Materials Technology, and others. Other suitable acrylates comprise acrylates/steareth-20 methacrylate copolymers such as made available by DOW Chemical (Aculyn) and homopolymers of acrylamide such as made available by Seppic & copolymers of acrylic acid, acrylamide and methacrylic acid esters, such as made available from Grain Processing, BASF, DOW Chemical, Lubrizol, AKZO Nobel, Goo Chemical, and others.

Polysaccharides that exhibit sufficient yield stress value are preferably selected from xanthan gum, various forms of carrageenan (preferably in combination with xanthan gum) and/or locust bean gum.

Synergy of the polymer with a clay material allows formulation of compositions with a yield stress value of at least 50 Pa that lead to a stable dispersion of chicken eggshells. Thus, in a preferred embodiment, the polymer is combined with mineral clay, preferably selected from sodium magnesium silicates, magnesium aluminium silicates and/or aluminium silicates. The boundary condition for choosing the desired polymer is the yield stress value: the yield stress value shall be at least 50 Pa to obtain a stable product. In practice, the yield stress value is preferably lower than 150Pa.

Preferably, compositions of the present invention comprise a polymer and preferably at a level of at least 0.1 % by weight, more preferably at least 0.2% by weight, most preferably at least 0.5% by weight of the composition and preferably at most 4% by weight, more preferably at most 2.5% by weight, most preferably at most 1.4% by weight of the composition.

Preferably, the composition of the present invention comprises one or more solvents. The solvent is preferably liquid and preferably selected from water, lower (C1-C4) alcohols and ketones, diols and triols. Examples include methanol, ethanol, propanol, isopropanol, butanol, isobutanol and glycerol, and water. Compositions of the present invention preferably comprise solvent at a level of at least 2% by weight, more preferably at least 10% by weight, most preferably at least 20% by weight and preferably at most 90% by weight of composition, more preferably at most 60% by weight of composition, most preferably at most 40% by weight of the composition.

Preferably, the composition of the present invention is aqueous. Preferably, the compositions of the present invention comprises water at a level of at least 2% by weight of composition, more preferably at least 10% by weight of composition, most preferably at least 20% by weight of the composition and preferably at most 90% by weight of composition, more preferably at most 60% by weight of composition, most preferably at most 40% by weight of the composition.

The pH of the composition comprising chicken eggshell particles may change over time and may drift towards higher pH values possibly due to dissolution of calcium carbonate. Preferably, according to the present invention, pH drifting of the compositions of the present invention is at least somewhat prevented as that may affect the chicken eggshell particles, in shape and/or level which might negatively affect the composition, particularly upon storage.

Compositions of the present invention preferably have a pH of higher than 7.5, more preferably of higher than 7.6, most preferably of higher than 7.7. For skin compositions, the pH of the composition is preferably lower than 9, more preferably lower than 8.7, most preferably lower than 8.5. Prior art skin products typically have a pH of between 5 and 7 corresponding to the skin pH.

Preferably, hard surface cleaning compositions have a pH of lower than 11, more preferably lower than 10, most preferably lower than 9.5.

In an embodiment, compositions of the present invention comprise a buffer to maintain the pH of the composition over time within the pH ranges indicated and prevent pH drift. Preferably, the compositions according to the invention comprise a phosphoric acid pH buffer. A preferred example is the combination of potassium dihydrogen phosphate and sodium hydroxide. For pharmacy application, the combination of phthalic acid and NAOH is preferred as buffer.

According to the invention, preferably, a drift in pH upon storage may be minimized, leading to compositions with chicken eggshell particles that are chemically and physically stably suspended in the composition over time.

Preferably, the composition of the invention, and in particular cream compositions, comprises one or more emollients. Emollients are oily substances that may be used in personal care & cosmetic creams. Preferably, the emollient is selected from:

- hydrocarbons (linear, branched, alicyclic) and/or aliphatic alcohols (linear, branched, alicyclic) that may originate from petrochemical origin, and preferably mixtures thereof;
- esters of vegetable fatty acids with a chain length of 6-24 carbon atoms, linear or branched, saturated or (poly)unsaturated, esterified with a suitable alcohol with a chain length of 1-24 carbon atoms, linear or branched, saturated or unsaturated;
- esters of polyhydric alcohols with vegetable fatty acid with a chain length of 6-24 carbon atoms, linear or branched, saturated or (poly)unsaturated;
- vegetable oils;
- waxes; and
- mixtures thereof.

Preferably, the emollient is present at a level of at least 1% by weight and preferably at most 20% by weight of the composition.

The composition of the present invention may further include optional ingredients such as one or more emulsifiers, foam boosters & stabilisers, refatting agents, irritation quenchers, fragrance, preservatives and water-soluble dyes.

Preferably, the compositions of the present invention are chemically and/or physically stable. We have found that use of the pH, as indicated, surprisingly contributes to the stability of the composition.

Chicken eggshell particles are preferably used in personal care products, cosmetic cream products (both scrub and polish), in household products, in chemical technical products and in medical products and the following preferred ranges can be indicated for the various applications.

The chicken eggshell particles of the invention may preferably be used for treatment of skin, preferably by exfoliating action. Exfoliating means removal of dead skin cells from the skin surface. Exfoliation can be accomplished by scrubbing the skin or by polishing the skin, polishing being the milder version of scrubbing and generally using smaller particle sizes in comparison.

The composition is preferably used as personal care scrub composition, for instance as bath and shower product. The chicken eggshell particles in such compositions are used for skin treatment, in particular to remove dead skin cells from the skin surface. The compositions preferably comprise from 3 to 6% by weight of chicken eggshells. Preferably, at least 50% by weight of the particle size of the chicken eggshell particles is preferably at least 297 $\mu$m and preferably at most 595 $\mu$m. Preferably, the composition comprises surfactant at a concentration of from 10 to 14%, preferably around 12%. Preferably, the pH of the composition is higher than 7.5 and lower than 8.5, more preferably around 8.0.

The composition is preferably used as cosmetic polishing cream composition. The chicken eggshell particles in such compositions are used for skin treatment, in particular to remove dead skin cells from the skin surface. Since these polishing cream compositions are gentler, they are preferably used to remove dead skin cells and treat the skin surface and can in particularly be used for treatment of the skin of the face. The cream is an oil-in-water or a water-in oil emulsion, preferably with a viscosity of 800-100,000 cPs Brookfield viscosity. Preferably, the composition comprises from 2 to 4% chicken eggshell particles. Preferably at least 50% by weight of the chicken egg particles have a particle size of preferably at least 88$\mu$m and preferably at most 297$\mu$m Preferably, the composition has a pH of higher than 7.5 and lower than 8.5, more preferably around 8.0.

The composition is preferably used as cosmetic scrub cream composition. The chicken eggshell particles in such compositions are used for skin treatment, in particular to remove dead skin cells from the skin surface. Thus, these compositions are used to remove dead skin cells and treat the skin surface. The cream is an oil-in-water or a water-in oil emulsions, preferably with a viscosity of 800-100,000 cPs Brookfield viscosity. Preferably, the composition comprises from 2 to 4% chicken eggshell particles. Preferably, at least 50% by weight of the chicken egg particles has a particle size of preferably at least 297$\mu$m and preferably at most 595$\mu$m. Preferably the composition has a pH of higher than 7.5 and lower than 8.5, more preferably around 8.0.

The composition is preferably as household product, more preferably a hard surface cleaner. The chicken eggshell particles can be used as abrasive for cleaning hard surfaces. Preferably, the composition comprises from 4 to 10% by weight of chicken eggshell particles. Preferably, at least 50% by weight of the chicken egg particles has a particle size of preferably at least 88$\mu$m and preferably at most 354 $\mu$m. Preferably, the composition comprises surfactant at a concentration of from 10 to 14% by weight, preferably around 12% by weight. The composition preferably has a pH of higher than 7.5 and preferably lower than 11, more preferably lower than 10, most preferably lower than 9.5.

The composition is preferably used as toothpaste. Chicken eggshell particles are used as abrasive for cleaning the teeth. The toothpaste is preferably a gel preparation with a preferred viscosity of 800-100,000 cPs Brookfield viscosity. The toothpaste preferably comprises from 3 to 7% chicken eggshell particles. Preferably, at least 50% by weight of the chicken egg particles have a particle size of preferably at least 88$\mu$m and preferably at most 354$\mu$m. The composition has a pH of preferably higher than 7.5 and preferably lower than 8.5, more preferably around 8.0. Preferably, the toothpaste has a pleasant mouth feel and sweetener such as glycerol, sorbitol.

The composition is preferably used as car polish. The chicken eggshell particles are used as abrasive. The car polish is preferably an oil-in-water or a water-in oil emulsion with a preferred viscosity of 800-100,000 cPs Brookfield viscosity. The car polish preferably comprises from 2 to 4% chicken eggshell particles. Preferably, at least 50% by weight of the chicken egg particles have a particle size of preferably at least 88$\mu$m to preferably at most 297$\mu$m The composition has a pH of preferably higher than 7.5 and preferably lower than 8.5, for instance around 8.0.

Other applications of chicken eggshells include a tablet aid (preferably a filler) for (pharmaceutical) tablets (with a preferred particle size of at least 50% by weight of from 100 to 300 $\mu$m), antacids to restore (and increase) the pH in the stomach (with a preferred particle size of at least 50% by weight of from 88 to 297$\mu$m) and in paint (with a preferred particle size of 50% by weight of from 88 to 210$\mu$m).

Preferably, chicken eggshells of the invention may be prepared as follows. The chicken egg content is separated from the chicken eggshell. The eggshell membrane may still be present on the surface of the chicken eggshells at this stage. The crude eggshells are washed with water, and subsequently dried in a stream of hot air, lowing the water content, for instance to a water content of less than 1%. This results in a water activity of less than 0.60 whereby micro-organisms are disabled to exhibit growth. The crude chicken eggshells are subsequently milled using a ball mill, hammer mill, disk mill or colloid mill (according to the invention a ball mill is preferred). The obtained granulate still contains eggshell membrane particles, that are removed by wind sifting. Wind sifting also enables to separate different fractions chicken eggshells. The equipment for wind sifting has been used elsewhere in industry, as for instance described in WO1998048950A1 (Metso Panelboard GmbH). Particular large fractions may be fed again into the milling equipment to further reduce the particle size. The present invention takes advantage of the difference in specific gravity of grinded chicken eggshell (density 2400-2800 kg/m3) and chicken eggshell membrane (1400 kg/m$^3$, the bulk weight is 400 kg/m$^3$).

After completion of the milling process and fractionation of the different chicken eggshell fractions, microbiological decontamination may be pursued using a suitable sterilisation agent, more particularly ozone, or eventually formaldehyde gas, whereby residual formaldehyde shall be less than 50 ppb.

Preferably, the chicken eggshells are sieved to ensure use of the desired particle size fraction for use in the composition of the invention. Preferably, standard sieves with US mesh are used according to ASTM C1360-06. Preferably, the resulting chicken eggshell fraction is subsequently free from dust and then packed.

Preferably, the composition of the present invention is prepared as follows:

The polymer material is preferably dispersed in the water under strong agitation, preferably with a propeller mixer.

Subsequently, surfactant, chicken eggshell particles, preservative, and/or solvent are added under stirring until a homogeneous dispersion is obtained.

The second step applies to the preparation of an emulsion and involves heating up the water phase (preferably to higher than 60°C and preferably lower than 80°C). Subsequently, the oil phase is mixed and may be heated, preferably to higher than 60°C and preferably lower than 80°C. The oil phase is preferably added to the water phase under mixing (preferably with a planetary mixer) and spontaneous emulsification will be observed. The pre-emulsion is subjected to mixing treatment, preferably with a rotor-stator mixer. The emulsion is cooled while stirring slowly, preferably with a planetary mixer. Any addition ingredients (such as fragrances, etc) may be added at 25°C. The pH may be adjusted to 7.5 to 8.0 with a 10% sodium hydroxide solution.

Chicken eggshell particles as used in the invention are particularly useful in view of the unique porosity and high purity. Without wishing to be bound by any theory, we believe that possibly upon storage of the compositions of the invention, the chicken egg shell particles may lose their sharp edges and become milder while maintaining the quality of the exfoliation activity. Although calcium carbonate is poorly soluble (the solubility product is $4,8 \times 10^{-9}$, equivalent to a solubility of 0,3% in water), the solubility may be more pronounced on the sharp edges of the shells, and therefore the harshness of the chicken egg shells is greatly reduced and not noticeable anymore after 1-2 weeks. Consequently, compositions of the invention are preferably stored for at least 2 weeks after production.

The present invention further relates to a method of treating the skin with the composition of the invention comprising the steps of:

a. providing a cleaning composition comprising surfactant (preferably anionic and/or nonionic) and chicken eggshell particles;
b. applying the composition to the skin;
c. adding water to wet the skin and develop a lather; and
d. rubbing the lather into the skin to remove dead skin cells from the skin surface.

As indicated, chicken eggshell particles exhibit unique porosity and high purity. Without wishing to be bound by any theory, we believe that upon application and using of the compositions of the invention on the skin, the chicken egg shell particles may slowly lose their sharp edges, possibly due to their porosity. Consequently, the efficacy of removing dead cells from the skin surface seems to slowly decrease during usage of the chicken eggshell particles in compositions of the invention, presenting a surprising and unique benefit, not provided by other agents.

Examples

Example 1

A personal care scrub composition for bath and shower with the following ingredients was prepared:

| Ingredients | level by weight) | INCI | function |
|---|---|---|---|
| Demineralized water | ad 100% | | |
| Texapon N70 | 12.5 | Sodium Laureth Sulfate, Aqua | surfactant |
| Dehyton K | 10.5 | Aqua, Cocamidopropyl Betaine | surfactant |
| Chicken eggshell * | 3.0 | Calcium Carbonate | exfoliating agent |
| Amidet N | 2.0 | PEG-4 Rapeseedamide, Aqua | foam stabiliser |
| Carbopol ETD2050 | 0.8 | Carbomer | flow control polymer |
| Preservative | qs | | |
| Perfume | qs | (solubilised in Polysorbate 20) | |
| Colouring Agent | qs | | |
| Sodium Hydroxide, 10% to pH=8.0 | | | |
| * with particle size of 354-420 μm, separated by sieving | | | |

The composition was prepared as follows: Carbopol ETD2050 is dispersed in water by adding the powdered polymer to water while strongly agitating with a propeller mixer. As soon as the polymer has been properly dispersed the surfactants are added and the mixture is stirred until homogeneous; Texapon N70 requires strong agitation. The viscosity will markedly increase. Subsequently chicken eggshells are added and mixed until homogeneous. Finally the preservative system, the dyes (optional) and the solubilised fragrance are added. The pH is adjusted to 7,5-8,0 while using a 10% sodium hydroxide solution. pH drift of the composition may be prevented by preferred inclusion of a pH buffer at a pH of 8.0.

The viscosity of the composition was 1,800 cPs (Brookfield viscosity,S4,20 rpm); the yield stress value was 65 Pa. The shower scrub composition was chemically and physically stable after 3 months at 40°C and, upon subsequently use, the composition provided excellent properties for removal of dead skin cells.

Example 2

A cosmetic polish cream composition with the following ingredients was prepared:

| Ingredients | level (% by weight) | INCI | function |
| --- | --- | --- | --- |
| Demineralized water | ad 100 | Aqua | solvent |
| Mineral oil, high viscous | 8.0 | Paraffinum Liquidum | emollient |
| Estol 1514 | 3.0 | Isopropyl Myristate | emollient |
| Brij 72 | 2.5 | Steareth-2 | nonionic emulsifier |
| Brij 721 | 2.5 | Steareth-21 | nonionic emulsifier |
| Lanette O | 3.0 | Cetearyl Alcohol | rheological additive |
| Chicken eggshells* | 1,8 | Calcium Carbonate | exfoliating agent |
| Rhodicare D | 0.6 | Xanthan Gum | flow control polymer |
| Perfume | qs | | |
| Preservative | qs | | |
| Sodium hydroxide, 10% to pH=8.0 | | | |
| * with particle size of 149-177μm, separated by sieving | | | |

The composition was prepared as follows: Rhodicare D is dispersed in water by adding the powdered polymer to water while strongly agitating with a propeller mixer. As soon as the polymer has been properly dispersed the chicken eggshell particles and the preservative system are added, and stirring is continued until a homogeneous dispersion has been obtained. The water phase of the emulsion is heated to 65-70°C. The oil phase is made by mixing the ingredients to 65-70°C, The oil phase is added to the water phase while stirring with a planetary mixer; spontaneous emulsification will be observed. The pre-emulsion is subjected to treatment with a rotor-stator mixer. The emulsion is cooled while stirring slowly with a planetary mixer. At 25°C the fragrance is added; solubilisation is not required, The pH is adjusted to 7,5-8,0 while using a 10% sodium hydroxide solution. pH drift of the composition may be prevented by preferred inclusion of a pH buffer in the water phase at a pH of 8.0.

The viscosity of the composition was 6,400 cPs (Brookfield viscosity,S4,20 rpm); the yield stress value was 55 Pa. The cream polish composition was chemically and physically stable after 3 months at 40°C and, upon subsequently use, the composition provided excellent properties for removal of dead skin cells.

Example 3

A cosmetic body scrub cream with the following ingredients was prepared:

| Ingredients | level (% by weight) | INCI | function |
| --- | --- | --- | --- |
| Demineralized water | ad 100 | Aqua | solvent |
| Emulgade SE | 6.0 | mixed emulsifier* | nonionic emulsifier |
| Sweet almond oil | 4.5 | Prunus Amygdalus Dulcis Oil | emollient |
| Pricerine 9099 | 3.0 | Glycerin | humectant |
| Lanette O | 3.0 | Cetearyl Alcohol | flow control |
| Cetiol V | 2.0 | Decyl Oleate | emollient |
| Cetiol 868 | 1.8 | Ethylhexyl Stearate | emollient |
| Chicken eggshell particles** | 1.8 | Calcium Carbonate | exfoliating agent |
| Rhodicare D | 0.5 | Xanthan Gum | flow control polymer |
| Perfume | qs | | |

(continued)

| Ingredients | level (% by weight) | INCI | function |
|---|---|---|---|
| Preservative | qs | | |
| Sodium Hydroxide, 10% | to pH=8.0 | | |

\* INCI: Glyceryl Stearate, Ceteareth-12, Ceteareth-20, Cetaryl Alcohol, Cetyl Palmitate

\*\* with particle size of 297-354 μm, separated by sieving

The composition was prepared as follows: Rhodicare D was dispersed in water by adding the powdered polymer to water while strongly agitating with a propeller mixer. As soon as the polymer has been properly dispersed glycerine, the chicken eggshell particles and the preservative system are added, and stirring is continued until a homogeneous dispersion has been obtained. The water phase of the emulsion is heated to 70-75°C. The oil phase is made by mixing the ingredients to 70-75°C, The oil phase is added to the water phase while stirring with a planetary mixer; spontaneous emulsification will be observed. The pre-emulsion is subjected to treatment with a rotor-stator mixer. The emulsion is cooled while stirring slowly with a planetary mixer. At 25°C the fragrance is added; solubilisation is not required, The pH is adjusted to 7,5-8,0 while using a 10% sodium hydroxide solution. pH drift of the composition may be prevented by preferred inclusion of a pH buffer at a pH of 8.0.

The viscosity of the composition was 8,200cPs (Brookfield viscosity, S5, 20rpm). The composition was chemically and physically stable after 3 months at 40°C and, upon subsequently use, the resulting body scrub composition provided excellent dead skin removing properties.

Example 4

A liquid abrasive hard surface cleaner, with the following ingredients was prepared:

| Ingredients | level (% by weight) | function |
|---|---|---|
| Water | 80.0 % | solvent |
| Fatty Alcohol Ethoxylate (n=3; Bio-Soft N61-6) | 10.0 % | nonionic |
| Ethanol | 4.0 % | solvent |
| Chicken eggshell particles* | 3.2 % | abrasive |
| Propylene Glycol n-Butyl Ether (Dowanol PnB) | 2.0 % | solvent |
| Sodium Magnesium Silicate (Laponite XLS) | 1.6 % | clay |
| Acrylates/Steareth-20 Methacrylate Crosspolymer (Aculyn 88) | 0.8 % | polymer |
| Preservative | qs | |
| Colorant | qs | |
| Fragrance (solubilised with POE-20 oleyl alcohol) | qs | |
| Sodium hydroxide solution | to pH 9.1 | |

\* with particle size of 149-210μm, separated by sieving

The composition was prepared as follows: Laponite XLS is dispersed in water by adding the powdered clay to water while strongly agitating with a propeller mixer. As soon as the clay has been properly dispersed the surfactant is added and the mixture is stirred until homogeneous and viscous. Aculyn 88 is subsequently added, developing good yield stress value. Subsequently, chicken eggshells are added and mixed until homogeneous. Finally the preservative system, the dyes (optional) and the solubilized fragrance are added. The pH is adjusted to 9.1 using a 10% sodium hydroxide solution.

The pH of the composition was 9.1; the viscosity of the composition was 2,200 cPs (Brookfield viscosity, S4, 20 rpm); the yield stress value was 71 Pa. The liquid abrasive cleaner composition was chemically and physically stable after 3 months at 40°C and, upon subsequently use, the composition provided excellent cleaning properties.

**Claims**

1.   Composition comprising from 1 to 30% by weight of chicken eggshell particles wherein at least 50% by weight of the chicken eggshell particles has a particle size of at least 74μm and at most 595μm.

2. Composition according to claim 1, wherein the pH of the composition is higher than 7.5 and lower than 9 and wherein the composition further comprises a polymer providing an external structure to the composition.

3. Composition according to claims 1-2, further comprising from 1-20% by weight of anionic surfactant and from 0.5-12% by weight of non-ionic surfactant with a ratio of anionic to nonionic surfactant of from 3:1 to 4:1.

4. Composition according to claims 1-3, further comprising egg shell membrane particles.

5. Composition according to claims 1-4, wherein the composition further comprises water at a level of from 2 to 90% by weight of composition.

6. Composition according to claims 1-5, wherein the composition further comprises emollient at a level of from 0.2% to 40% by weight of the composition.

7. Method of preparing a composition comprising chicken eggshells and polymer material, wherein the chicken eggshell is ball-milled to particles which are subsequently dispersed in the composition comprising polymer material and wherein the pH is subsequently set at higher than 7.5.

8. Method according to claim 7, wherein at least 50% by weight of the chicken eggshell particles has a particle size of at least $74\mu$m and at most $595\mu$m.

9. Method of treating the skin by removing dead skin cells from the skin surface with chicken eggshell particles.

10. Method according to claim 9, wherein the chicken eggshell particles are first ball-milled.

11. Method according to claims 9-10, wherein the skin treatment comprises the steps of:

    a. providing a cleaning composition comprising surfactant and chicken eggshell particles;
    b. applying the composition to the skin;
    c. adding water to wet the skin and develop a lather; and
    d. rubbing the lather into the skin to remove dead skin cells from the skin surface.

12. Method of preparing chicken eggshell particles by washing and ball-milling the eggshells, wind sifting and sterilizing the resulting chicken eggshell particles, and subsequently sieving the chicken eggshell particles into fractions.

13. Method of preparing a composition comprising chicken eggshell particles dispersed in an aqueous composition comprising polymer, wherein the polymer is mixed with water, the chicken eggshell particles are added under stirring and the pH of the composition is set at higher than 7.5.

14. Chicken eggshell particles for use in treatment of skin.

15. Chicken eggshell particles for use according to claim 12, wherein at least 50% by weight of the chicken eggshell particles has a particle size of at least $74\mu$m and at most 595 $\mu$m.

Europäisches
Patentamt

European
Patent Office

Office européen
des brevets

# EUROPEAN SEARCH REPORT

Application Number

EP 13 15 7848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | "Egg Shell Powder" In: Tara E. Gottschalck, Halyna P. Breslawec: "International Cosmetic Ingredient Dictionary and Handbook", 2012, PErsonal Care Products Council, Washington D.C., XP002711932, vol. 1, page 1107, * "Egg Shell Powder" * ----- | 1-6 | INV. A61Q19/10 A61K8/98 C09K3/14 C11D3/12 C11D3/14 A61K8/73 A61K8/81 |
| X | CN 101 191 047 A (SHANGHAI YUSHENG TECHNOLOGY CO [CN]) 4 June 2008 (2008-06-04) | 1,5 | |
| Y | * abstract; examples * ----- | 1-6 | |
| X | DATABASE WPI Week 200858 Thomson Scientific, London, GB; AN 2008-J84147 XP002711933, "Friction agent from eggshells does not contain heavy metal impurities, such as mercury, lead and manganese and will not produce poisonous side-effect in the human body", & CN 101 191 047 A (SHANGHAI YUSHENG SCI & TECHNOLOGY CO LTD) 4 June 2008 (2008-06-04) | 1,5 | |
| Y | * abstract * ----- -/-- | 1-6 | |

TECHNICAL FIELDS
SEARCHED (IPC)

A61Q
A61K
C09K
C11D

~~The present search report has been drawn up for all claims~~

| Place of search | Date of completion of the search | Examiner |
|---|---|---|
| Munich | 29 August 2013 | Sala-Jung, Nathalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# EP 2 774 655 A1

**EUROPEAN SEARCH REPORT**

Europäisches Patentamt

European Patent Office

Office européen des brevets

Application Number

EP 13 15 7848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 200166<br>Thomson Scientific, London, GB;<br>AN 2001-587621<br>XP002711934,<br>"Mouth washing constitution containing solid component",<br>& KR 2001 0036526 A (SHIN K H)<br>7 May 2001 (2001-05-07) | 1,5 | |
| Y | * abstract * | 1-6 | |
| Y | US 6 344 217 B1 (RUEPP MICHEL O [DE])<br>5 February 2002 (2002-02-05)<br>* column 1, line 25 - line 38 *<br>* column 1, line 39 - column 2, line 67 * | 1-6 | |
| X | CN 101 665 619 A (UNIV SHANGHAI)<br>10 March 2010 (2010-03-10) | 1 | |
| Y | * abstract *<br>* page 3; examples 1,6 * | 1-6 | |
| X | DATABASE WPI<br>Week 201334<br>Thomson Scientific, London, GB;<br>AN 2013-G05434<br>XP002711935,<br>"Nutrient soup for pig comprises pepper powder, borneol powder, gypsum powder, and dry egg shell powder",<br>& CN 102 871 003 A (CHEN J)<br>16 January 2013 (2013-01-16) | 1,5 | TECHNICAL FIELDS SEARCHED (IPC) |
| Y | * abstract * | 1-6 | |
| Y | GB 1 474 389 A (PYRENE CHEM SERVICES LTD)<br>25 May 1977 (1977-05-25)<br>* page 1, line 31 - line 42 *<br>* page 2 * | 1-6 | |

-/--

~~The present search report has been drawn up for all claims~~

| Place of search | Date of completion of the search | Examiner |
|---|---|---|
| Munich | 29 August 2013 | Sala-Jung, Nathalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

14

Europäisches
Patentamt

European
Patent Office

Office européen
des brevets

## EUROPEAN SEARCH REPORT

Application Number

EP 13 15 7848

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP H04 187615 A (ISHIZUKA YOZO) 6 July 1992 (1992-07-06) * abstract * ----- | 1-6 | |
| Y | US 2004/266645 A1 (ALBRECHT HARALD [DE] ET AL) 30 December 2004 (2004-12-30) * paragraph [0009] - paragraph [0014] * * paragraph [0020] - paragraph [0022]; examples * ----- | 1-6 | |
| Y | EP 1 674 134 A1 (BEIERSDORF AG [DE]) 28 June 2006 (2006-06-28) * paragraph [0015]; examples * ----- | 1-6 | |
| Y | US 2007/179078 A1 (COLLIN JENNIFER R [US] ET AL) 2 August 2007 (2007-08-02) * paragraphs [0004], [0018]; examples 8,12-14 * ----- | 1-6 | |

TECHNICAL FIELDS
SEARCHED        (IPC)

The present search report has been drawn up for all claims

| Place of search | Date of completion of the search | Examiner |
|---|---|---|
| Munich | 29 August 2013 | Sala-Jung, Nathalie |

EPO FORM 1503 03.82 (P04C01)

Europäisches
Patentamt

European
Patent Office

Office européen
des brevets

Application Number

EP 13 15 7848

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

## LACK OF UNITY OF INVENTION
### SHEET B

Application Number

EP 13 15 7848

The Search Division considers that the present European patent application does not comply with the requirements of unity of invention and relates to several inventions or groups of inventions, namely:

1. claims: 1-6

   Composition comprising from 1 to 30% by weight of chicken eggshell particles wherein at least 50% by weight of the chicken eggshell particles has a particle size of at least 74 microns and at most 595 microns

   ---

2. claims: 7, 8

   Method of preparing a composition comprising chicken eggshells and polymer material, wherein the chicken eggshell is ball-milled to particles which are subsequently dispersed in the composition comprising polymer material and wherein the pH is subsequently set at higher than 7.5

   ---

3. claims: 9-11, 14, 15

   Chicken eggshell particles for use in treatment of skin, and Method of treating the skin by removing dead skin cells from the skin surface with chicken eggshell particles

   ---

4. claim: 12

   Method of preparing chicken eggshell particles by washing and ball-milling the eggshells, wind sifting and sterilizing the resulting chicken eggshell particles, and subsequently sieving the chicken eggshell particles into fractions.

   ---

5. claim: 13

   Method of preparing a composition comprising chicken eggshell particles dispersed in an aqueous composition comprising polymer, wherein the polymer is mixed with water, the chicken eggshell particles are added under stirring and the pH of the composition is set at higher than 7.5

   ---

## ANNEX TO THE EUROPEAN SEARCH REPORT
## ON EUROPEAN PATENT APPLICATION NO.

EP 13 15 7848

This annex lists the patent family members relating to the patent documents cited in the above-mentioned European search report.
The members are as contained in the European Patent Office EDP file on
The European Patent Office is in no way liable for these particulars which are merely given for the purpose of information.

29-08-2013

| Patent document cited in search report | | Publication date | Patent family member(s) | | Publication date |
|---|---|---|---|---|---|
| CN 101191047 | A | 04-06-2008 | NONE | | |
| CN 101191047 | A | 04-06-2008 | NONE | | |
| KR 20010036526 | A | 07-05-2001 | NONE | | |
| US 6344217 | B1 | 05-02-2002 | US | 6344217 B1 | 05-02-2002 |
| | | | US | 2002090400 A1 | 11-07-2002 |
| | | | US | 2007122489 A1 | 31-05-2007 |
| | | | US | 2010151040 A1 | 17-06-2010 |
| CN 101665619 | A | 10-03-2010 | NONE | | |
| CN 102871003 | A | 16-01-2013 | NONE | | |
| GB 1474389 | A | 25-05-1977 | NONE | | |
| JP H04187615 | A | 06-07-1992 | NONE | | |
| US 2004266645 | A1 | 30-12-2004 | DE | 10157541 A1 | 12-06-2003 |
| | | | EP | 1453480 A1 | 08-09-2004 |
| | | | JP | 2005513022 A | 12-05-2005 |
| | | | US | 2004266645 A1 | 30-12-2004 |
| | | | WO | 03043599 A1 | 30-05-2003 |
| EP 1674134 | A1 | 28-06-2006 | AT | 421360 T | 15-02-2009 |
| | | | DE | 102004062771 A1 | 22-06-2006 |
| | | | EP | 1674134 A1 | 28-06-2006 |
| | | | ES | 2319308 T3 | 06-05-2009 |
| US 2007179078 | A1 | 02-08-2007 | NONE | | |

EPO FORM P0459

For more details about this annex : see Official Journal of the European Patent Office, No. 12/82

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050170979 A
- US 20030133900 A, McLaughlin
- JP 4169513 B
- JP 4187616 B
- WO 1998048950 A1

**Non-patent literature cited in the description**

- **ZHAO ; CHI.** *Biotechnology,* 2009, vol. 8 (2), 254-258
- **SCHWARTZ ; PERRY.** Surface Active Agents. Interscience, 1949, vol. 1
- **SCHWARTZ ; PERRY ; BERCH.** SURFACE ACTIVE AGENTS. Interscience, 1958, vol. 2
- McCutcheon's Emulsifiers and Detergents. Manufacturing Confectioners Company
- **H. STACHE.** Tenside-Taschenbuch. Carl Hauser Verlag, 1981
- **RIEGER.** Surfactant Encyclopaedia. Allured Publishing, 1996